# EUROPEAN PATENT APPLICATION

(11) **EP 0 937 774 A1**
(43) Date of publication of application: **25.08.1999**
(21) Application number: 99610011.1
(22) Date of filing: 18.02.1999
(51) Int. Cl.: C12N 9/02, C12N 1/20, C12N 15/01, C12P 7/18, C12P 7/26, C12P 7/42, A23L 1/03

(54) **Lactic acid bacteria having a reduced pyruvate dehydrogenase activity and use thereof**

(30) Priority: 20.02.1998 DK 24198
(71) Applicant: Chr. Hansen A/S, 2970 Hoersholm (DK)
(72) Inventor: Nilsson, Dan, 3060 Espergaerde (DK); Henriksen, Claus Maxel, 2100 Copenhagen (DK)
(74) Representative: Plougmann, Vingtoft & Partners A/S

(57) **Abstract**

Lactic acid bacterial strain including *Lactococcus lactis* which are defective in their pyruvate dehydrogenase complex and/or in their lactate dehydrogenase and methods of isolating such strains are provided. The strains are useful in the production of food products or in the manufacturing of compound such as diacetyl, acetoin and α-acetolactate and as a components of food starter cultures.

## Description

### FIELD OF INVENTION

The present invention relates to the field of lactic acid bacterial starter cultures and in particular there is provided means for metabolically engineering such bacteria to obtain mutants or variants hereof which, when they are used in the manufacturing of food products produce increased amounts of desirable metabolites or reduced amounts of less desirable metabolites.

### TECHNICAL BACKGROUND AND PRIOR ART

Lactic acid bacteria are used extensively as starter cultures in the food industry in the manufacturing of fermented products including milk products such as e.g. yoghurt and cheese, meat products, bakery products, wine and vegetable products. *Lactococcus* species including *Lactococcus lactis* are among the most commonly used lactic acid bacteria in dairy starter cultures. However, several other lactic acid bacteria such as *Leuconostoc* species, *Pediococcus* species, *Lactobacillus* species and *Streptococcus* species are also commonly used in food starter cultures.

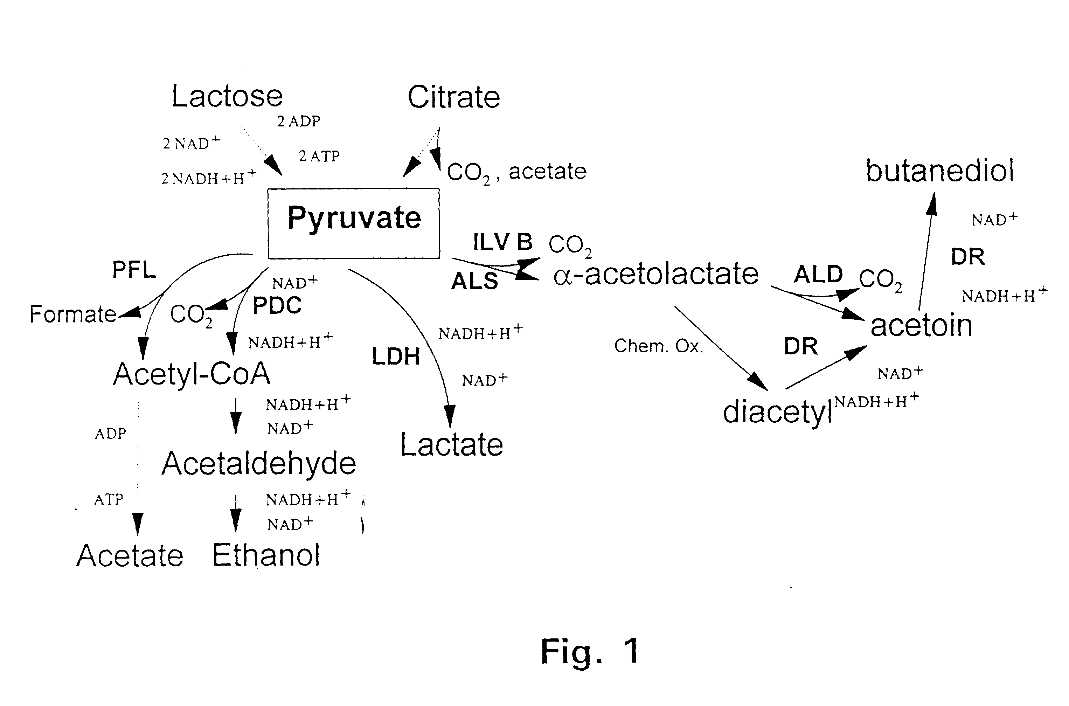

When a lactic acid bacterial starter culture is added to milk or any other food product starting material under appropriate conditions, the bacteria grow rapidly with concomitant conversion of citrate, lactose or other sugar compounds into lactic acid/lactate and possibly other acids including acetate, resulting in a pH decrease. In addition, several other metabolites are produced during the growth of lactic acid bacteria (Fig. 1). These metabolites include ethanol, formate, acetaldehyde, α-acetolactate, acetoin, diacetyl, and 2,3 butylene glycol (butanediol). Among these metabolites, diacetyl is an essential flavour compound which is formed during fermentation of the species of e.g. *Lactococcus, Leuconostoc* and *Lactobacillus.* Diacetyl is formed by an oxidative decarboxylation (Fig. 1) of α-acetolactate which is formed by the action of α-acetolactate synthetase (ALS) from two molecules of pyruvate.

Pyruvate is a key intermediate of several lactic acid bacterial metabolic pathways including the citrate metabolism and the degradation of lactose or other sugars to lactate. A major part of the pyruvate pool in lactic acid bacteria is converted to lactate by the enzyme lactate dehydrogenase (LDH).

Part of the pyruvate pool is converted to diacetyl, acetoin and butanediol via the intermediate compound α-acetolactate. The pool of pyruvate in the cells is critical for the flux through the metabolic pathway leading to diacetyl, acetoin and butanediol due to the low affinity of α-acetolactate synthetase for pyruvate.

Furthermore, a part of the pyruvate is converted to the intermediate compound acetyl-CoA which in turn is converted to acetaldehyde, ethanol or acetate. Acetyl-CoA is an important precursor in other biosynthetic reactions, e.g. lipid biosynthesis.

Under anaerobic conditions, acetyl-CoA is formed from pyruvate by pyruvate formate lyase (PFL). However, this enzyme is irreversibly inactivated under aerobic conditions. However, under aerobic conditions acetyl-CoA is formed from pyruvate by the pyruvate dehydrogenase complex (PDC). This complex comprises three catalytic and two regulatory enzymes (Hinman et al. 1981). PDC has a very low activity under anaerobic conditions due to inhibition by high NADH/NAD⁺ ratios (Snoep et al. 1993) and requires the presence of lipoic acid (or lipoamide) as a co-factor to be functional under aerobic conditions. However, most lactic acid bacteria do not have a *de novo* synthesis of lipoic acid and the presence of lipoic acid in the growth medium is therefore required for the PDC to be active.

Additionally, acetyl-CoA can be produced in lactic acid bacteria from acetate under aerobic as well as under anaerobic conditions. However, this step requires energy in the form of ATP.

As mentioned above, the metabolites which are derived from α-acetolactate are desirable compounds both in fermented food products such as dairy products and in flavour product for the food industry. However, the level of production of such metabolites by lactic acid bacteria are generally much higher under aerobic conditions as compared to the production under anaerobic conditions and thus, there is an industrial interest in having lactic acid bacterial strains which under aerobic conditions has an enhanced production of α-acetolactate and compounds derived therefrom including diacetyl.

It is therefore one objective of the present invention to provide lactic acid bacterial strains having under aerobic conditions an enhanced production of the above metabolites.

Recent studies have shown that when *L. lactis* is lacking the enzyme LDH more pyruvate is directed towards acetoin and butanediol via α-acetolactate, possibly resulting in increased formation of diacetyl (Platteeuw et al., 1995; Gasson et al., 1996). However, such modified strains will still produce acetate and ethanol.

Overproduction of α-acetolactate synthetase in *Lactococcus lactis* as another approach for metabolically engineering lactic acid bacteria to produce increased amounts of diacetyl has been disclosed by Platteeuw et al. 1995.

As it is mentioned above (and illustrated in Fig. 1), the multi-enzyme pyruvate dehydrogenase complex is involved in the metabolic pathways leading to the formation of the above metabolites. Whereas Anders & Jago (1970) have suggested to block the Pdc activity in lactic acid bacteria by the addition of oleic acid to the cultivation medium as a means of modulating flavour compound production under aerobic conditions in i.e. *Lactococcus* spp., the prior art is not aware of lactic acid bacteria which are metabolically engineered so as to provide mutant bacteria having a reduced Pdc activity as a means of providing lactic acid bacteria which under aerobic conditions possess improved characteristics with respect to production of acetoin and/or diacetyl.

As it is mentioned above, wild-type strains of lactic acid bacteria such as strains of *Lactococcus* and *Streptococcus* including as examples *Lactococcus lactis* and *Streptococcus thermophilus* strains require either acetate or lipoic acid for growth under aerobic conditions. The inventors have used these inherent characteristics as the basis for developing a novel and simple method for the isolation of Pdc defective lactic acid bacterial mutants (Pdc) as it will be described in the following.

Additionally, having such a method allowing the selection of Pdc defective lactic acid bacterial mutants at hand it is possible to provide further mutated cells which in addition to being Pdc⁻ are mutated in one or more genes involved in the citrate/sugar metabolic pathways such as e.g. the *Idh* gene coding for lactate dehydrogenase (LDH) or the *pfl* gene coding for pyruvate formate lyase (PFL), so as to provide a variety of metabolically engineered lactic acid bacteria having highly desirable improved characteristics with respect to metabolite production.

The above findings have thus opened up for a novel approach for providing useful metabolically engineered lactic acid bacterial starter cultures which approach is based on relatively simple classical random mutagenesis methods or screening for spontaneously occurring mutants and which does not involve in vitro genetic engineering.

### SUMMARY

Accordingly, the invention provides in a first aspect a lactic acid bacterial strain which under aerobic conditions has a specific pyruvate dehydrogenase activity as determined by the method according to Garrigues et al. (1997) which is reduced by at least 10% relative to that of the *Lactococcus lactis* subsp. *lactis* strain CHCC373 deposited under accession No. DSM 12015 grown under the same conditions.

In a further aspect, the invention relates to a method of isolating such a strain, comprising the steps of providing a lactic acid bacterial parent strain which under aerobic conditions has a growth requirement for lipoic acid and which is capable of growing in the absence of acetate, cultivating said strain on a first medium containing lipoic acid and acetate and then on a second medium which is the same as the first medium but without acetate and selecting a strain that is capable of growing on the first medium but not on the second medium.

In a still further aspect, the present invention relates to a method of isolating a lactic acid bacterial DNA sequence coding for at least one gene product of the pyruvate dehydrogenase complex, comprising isolating a lactic acid bacterial *pdc* mutant, and identifying in a lactic acid bacterium a gene that can restore the defective Pdc activity upon transformation of said mutant and isolating the gene.

The invention pertains in another aspect to a method of constructing a lactic acid bacterial strain according to the invention, the method comprising replacing a wild-type *pdc* gene of a lactic acid bacterium by the mutated gene as mentioned above.

In further aspects, the invention relates to a method of producing a food product, comprising adding to the food product starting materials a culture of the lactic acid bacterial strain according to the invention, and a method of producing a lactic acid bacterial metabolite, comprising cultivating a lactic acid bacterial strain according to the invention under conditions where the metabolite is produced, and isolating the metabolite from the culture.

There is also provided a lactic acid bacterial starter culture composition comprising a strain according to the invention.

### DETAILED DISCLOSURE OF THE INVENTION

Thus, it is an important objective of the present invention to provide a lactic acid bacterial strain which under aerobic conditions has a specific pyruvate dehydrogenase activity which is reduced by at least 10% relative to that of the *Lactococcus lactis* subsp. *lactis* strain CHCC373 deposited with the Deutsche Sammlung von Mikroorganismen und Zellkulturen, Mascheroder Weg, 1b, D - 38124 Braunschweig on 17 February 1998 under the accession No. DSM 12015, grown under the same conditions.

As used herein, the expression "lactic acid bacterium" designates gram positive, microaerophilic or anaerobic bacteria which ferment sugar with the production of acids including lactic acid as the predominantly produced acid, acetic acid, formic acid and propionic acid. The industrially most useful lactic acid bacteria are found among *Lactococcus* species, *Streptococcus* species, *Lactobacillus* species, *Leuconostoc* species, *Pediococcus* species. Also the strict anaerobes belonging to the genus *Bifidobacterium* is in generally included in the group of lactic acid bacteria.

In useful embodiments, the lactic acid bacterial strain of the present invention is one which under aerobic conditions has a specific pyruvate dehydrogenase activity which is reduced by at least 20% relative to that of the deposited strain CHCC373 when grown under the same conditions. It is, however preferred that the pyruvate dehydrogenase activity is reduced even more such as by at least 30% e.g at least 40% including at least 50%, such as by at least 60% including by at least 70%, e.g. by at least 80% including by at least 90% relative to that of the deposited strain CHCC373 when grown under the same conditions.

It will be understood that the pyruvate dehydrogenase activity of the bacterial strain according to the invention is determinated according to Garrigues et al. (1997), a spectrophotometric assay which specifically measures the NADH production of the pyruvate dehydrogenase complex by coupling NADH to the reduction of a tetrazolium dye via the intermediate electron carrier lipoamide dehydrogenase. Thus, in useful embodiments of the invention, the bacterial strain is one which under aerobic conditions has a specific pyruvate dehydrogenase activity which is at the most 12.0 nmoles NADH min⁻¹ mg-¹ protein such as at the most 10.0 nmoles NADH min⁻¹ mg⁻¹ protein including at the most 8.0 nmoles NADH min⁻¹ mg protein e.g. at the most 6.0 nmoles NADH min⁻¹ mg⁻¹ protein. In particularly useful embodiments, the bacterial strain is one which under aerobic conditions has a specific pyruvate dehydrogenase activity which is at the most 4.0 nmoles NADH min⁻¹ mg protein such as at the most 2.0 nmoles NADH min⁻¹ mg⁻¹ protein.

As a result of the reduced pyruvate dehydrogenase activity of the bacterial strain according to the present invention, said strain is capable of converting, under aerobic conditions, a substantial proportion of the intracellular pyruvate pool to α-acetolactate and further to one or more of the metabolites which can be formed from this intermediate compound including acetoin, butanediol and/or diacetyl which latter compound can be formed by chemically oxidizing α-acetolactate. Thus, in one preferred embodiment, the bacterial strain has under aerobic conditions an increased production of α-acetolactate and the metabolites derived therefrom, of at least 20% relative to that of the deposited strain CHCC373, more preferably at least 30%. In even more preferred embodiments, this production is increased by at least 40%, such as at least 50% or even at least 60% relative to that of the CHCC373 strain.

As it is shown in the below Examples, the increase of production of α-acetolactate is significant. Thus, a bacterial strain according to the invention may when grown under aerobic conditions on reconstituted skim milk (RSM), have a production of α-acetolactate which is at least 20 ppm such as 30 ppm. In useful embodiments the production of α-acetolactate is at least 40 ppm such as at least 50 ppm.

Several of these characteristics may be desirable for specific purposes. In the production of a food product it may thus be advantageous that the strain produces higher amounts of α-acetolactate-derived aroma or flavour compounds, whereas a reduced production of acetate or ethanol can be highly desirable, in particular in the production of dairy products. In useful embodiments, the bacterial strain according to the invention has, when grown on RSM under aerobic conditions, a production of metabolites such as ethanol or acetate which is at the most 80% relative to that of the deposited *Lactococcus lactis* subsp. *lactis* strain CHCC373, more preferably at the most 70% and in particular at the most 60% including at the most 50%, such as at the most 40%, e.g. at the most 30%, such as at the most 20%, including 10% relative to that of CHCC373 strain. Besides being useful in the manufacturing of a food product, a mutant strain overproducing α-acetolactate-derived metabolites can also be used in the production of the metabolites as such.

In useful embodiments, the bacterial strain according to the invention has, under the above conditions, a production of acetate which is at the most 250 ppm including at the most 200 ppm, e.g. at the most 150 ppm. In a particularly useful embodiment, the bacterial strain has, under the above conditions, a reduced production of acetate which is at the most 100 ppm including at the most 50 ppm such as at the most 25 ppm, including at the most 10 ppm.

In one important embodiment of the present invention, the lactic acid bacterial strain is one which can be derived by selecting a mutant of a wild-type strain of a lactic acid bacterium which under aerobic conditions and in the absence of acetate has a growth requirement for lipoic acid, said mutant having, under the same conditions, a growth requirement for acetate regardless of the presence of lipoic acid.

It is assumed that a wild-type strain of a lactic acid bacterium is not capable of growth under aerobic conditions in the absence of lipoic acid which, as mentioned above, is an essential co-factor for the activity of the pyruvate dehydrogenase complex (PDC). This is due to the fact that this complex does not generate acetyl-CoA. Therefore, under aerobic conditions, the wild-type lactic acid requires acetate as an alternative source of acetyl-CoA. Thus, under aerobic conditions, a bacterial mutant strain derived from the above wild-type lactic acid bacterial strain, which requires acetate regardless of the presence of lipoic acid, is assumed to have a defect in a gene involved in the PDC and is therefore of the phenotype Pdc. In addition to its Pdc phenotype, such a mutant strain can be distinguished from its parent strain in one or more characteristics as mentioned above. Examples of such strains are the *Lactococcus lactis* subspecies *lactis* TDH-1 and TDH-2 strains as deposited with the Deutsche Sammlung von Mikroorganismen und Zellkulturen, Mascheroder Weg, 1b, D - 38124 Braunschweig on 11 February 1998, under the accession No. DSM 11996 and No. DSM 11997, respectively.

As mentioned above, wild-type strains of lactic acid bacteria have a requirement for lipoic acid when grown under aerobic conditions. However, such bacterial strains do not have a *de novo* synthesis of lipoic acid, and lipoic acid therefore needs to be present in the medium for the PDC to be active. Milk and some other complex media contain lipoic acid. Variations in the content of lipoic acid in milk might have an influence on the activity of the pyruvate dehydrogenase complex and thereby on the fermentation product formation and on the reproducibility of the production of fermented milk products.

Accordingly, it is expected that the growth and fermentation product formation of Pdc-defective strains, which do not require lipoic acid for growth under aerobic conditions, are independent of the content of lipoic acid in milk, and that the reproducibility of the production of fermented milk products can be increased by applying these strains.

One useful embodiment of the present invention is one where the Pdc defective mutant is mutated in at least one further gene involved in the pyruvate metabolism. In one useful specific embodiment of the invention, the mutant is one, which is defective in the Pdc and Ldh activity. In addition to its Pdc⁻ Ldh⁻ phenotype, such a mutant can be distinguished from a wild-type lactic acid bacterium or its Pdc defective parent strain in that, when it is cultivated under aerobic conditions, a larger part of the pyruvate pool is directed towards α-acetolactate, acetoin and/or diacetyl, due to the reduced capability of the mutant strain to convert lactose to lactic acid/lactate, caused by the defect in the *Idh* gene.

In a further aspect, the invention relates to a mutant or a variant of the above Pdc Ldh mutant lactic acid bacterium which has an additional defect in the gene coding for pyruvate formate lyase (PFL). Such a mutant or variant with the phenotype Pdc Ldh⁻ Pfl⁻, is not capable of growing under aerobic condition without acetate and not at all under anaerobic conditions.

In the present context, the term "variant" refers to a lactic acid bacterium which has been modified in any other characteristic than those related to the pyruvate metabolism. By way of example such a modification can be resistance to an antibiotic or bacteriocin, modified growth requirements or nutrient requirements.

It will be understood that the above mutants can be starting materials for providing further lactic acid bacterial mutants or variants. One example is the above Pdc⁻ Ldh⁻ Pfl⁻ mutant which can be utilized as a parent strain for selecting a mutant which has regained its Pfl activity and thus is capable of growing anaerobically regardless of the presence of acetate, i.e. having the phenotype Pdc⁻ Ldh⁻ Pfl⁺.

Another example of the use of any of the above mutant strains is to provide further mutated strain where the mutation results in overproduction and/or enhanced activity of an enzyme, the activity of which can be limiting for a pathway in which a NADH dependent oxidoreductase is involved. Such an overproduction or enhanced activity can e.g. be of the α-acetolactate synthetase (ALS), the increased production or activity of which would in turn result in an increased production of substrate for diacetyl reductase. Alternatively, the mutation may result in the above enzyme having an increased activity.

NADH dependent oxidoreductases require a substrate. Thus, as an example, acetoin is the substrate for the oxidoreductase diacetyl reductase (see Fig. 1). Accordingly, it is contemplated that any of the above mutants can be used for selecting a mutant which does not grow even if the oxidoreductase substrate such as acetoin is added to the medium. Assumingly such a mutant will have a defect in one or more of its oxidoreductases e.g. diacetyl reductase.

In accordance with the invention, the above mutants can be of any lactic acid bacterial species selected from a *Lactococcus* species, a *Lactobacillus* species, a *Leuconostoc* species, a *Pediococcus* species, a *Streptococcus* species and a *Bifidobacterium* species. One preferred species is *Lactococcus lactis,* including *Lactococcus lactis* subspecies *lactis* and *Lactococcus lactis* subspecies *lactis* biovar *diacetylactis.*

In a further aspect, the invention relates to a method of selecting and isolating a spontaneously occurring Pdc⁻ strain, comprising the steps of providing a lactic acid bacterial parent strain which under aerobic conditions has a growth requirement for lipoic acid and which is capable of growing in the absence of acetate, cultivating said strain on a first medium containing lipoic acid and acetate and on a second medium which is the same as the first medium but without acetate and selecting a strain that is capable of growing on the first medium but not on the second medium.

It is assumed that such a mutant strain has a defect in at least one gene coding for a polypeptide involved in the PDC implying that overall activity of the complex is reduced which may be due to several modification such as a reduced production of a catalytic enzyme, an increased production of a regulatory enzyme and/or a modification of an enzyme of the complex leading to a modified specific activity or to the production of an enzyme in an at least partially inactive form. This assumption can be affirmed by testing the selected mutant for significantly lower production of ethanol and/or acetate or alternatively, a reduced pyruvate dehydrogenase activity.

Although the lactic acid bacterial mutant strain of the invention can be provided by selecting a spontaneously occurring mutant in accordance with the screening procedure as described above, it will be understood that it is possible to provide the Pdc⁻ mutant by subjecting the wild-type lactic acid bacterial strain to a mutagenization treatment prior to the selection of a mutant. This method provides in a first step the provision of a wild-type lactic acid bacterium having under aerobic conditions a requirement for acetate or lipoic acid, followed by subjecting the bacterium to a mutagenization treatment. In accordance with the invention, suitable mutagens include conventional chemical mutagens and UV light. Thus, as examples, a chemical mutagen can be selected from (i) a mutagen that associates with or become incorporated into DNA such as a base analogue, e.g. 2-aminopurine or an interchelating agent such as ICR-191, (ii) a mutagen that react with the DNA including alkylating agents such as nitrosoguanidine or hydroxylamine, or ethane methyl sulphonate (EMS).

As an alternative to one presently preferred method of providing the Pdc⁻ mutant by random mutagenesis, it is also possible to provide such a mutant by site-directed mutagenesis, e.g. by using recombinant DNA techniques.

The lactic acid bacterial wild-type parent strain can be selected from any industrially suitable lactic acid bacterial species, i.e. the strain can be selected from the group consisting of a *Lactococcus* species, a *Lactobacillus* species, a *Leuconostoc* species, a *Pediococcus* species and a *Streptococcus* species. In particular useful embodiments, the lactic acid bacterium is a *Lactococcus lactis* or a *Streptococcus thermophilus.* Examples of presently preferred lactic acid bacteria are *Lactococcus lactis* subspecies *lactis* and *Lactococcus lactis* subspecies *lactis* biovar *diacetylactis.*

Importantly, the Pdc⁻ strain according to the invention can also be used as a parent strain for isolating mutants having further useful enzymatic defects as it will be described in the following.

As mentioned above, lactate dehydrogenase (LDH) is, another enzyme which in lactic acid bacteria contribute to the consumption of the pyruvate pool, the activity of the enzyme predominantly resulting in the production of lactate. It is contemplated that the metabolic flux towards α-acetolactate and metabolites derived from this intermediate could be further increased by providing a mutant strain which carries both a defect in the Pdc and Ldh.

Therefore, one example of a useful method for isolating and selecting a lactic acid bacterium which carries both a defect in Pdc and Ldh, i.e. having the Pdc⁻ Ldh⁻ phenotype, comprises in a first step the provision of a Ldh defective strain. In this context, one preferred strain is the strain *Lactococcus lactis* subspecies *lactis* DN224 deposited under the accession No. DSM 11037. This strain has the Pfl⁺ Ldh⁻ Ang⁺ (Ang: capable of growth anaerobically) phenotype and is derived from a parent strain which is incapable of growing under anaerobically conditions in an acetate-containing medium, i.e. having the Pfl⁻ Ldh⁻ Ang⁻ phenotype, said mutant strain being capable of growth under the same conditions. Thus, having provided the Ldh defective strain, the method comprises in a further step a selection of a strain from this Ldh defective bacterium which under aerobic conditions has a growth requirement for acetate regardless of the present of lipoic acid.

In one presently preferred embodiment of the above method, the Ldh defective parent strain is subjected to a mutagenization treatment as described above prior to selection of a mutant strain with a Pdc⁻ Ldh⁻ phenotype.

In another example of a useful method for isolating a double mutant with the Pdc⁻ Ldh⁻ phenotype, the parent strain for such a double mutant is the strain *Lactococcus lactis* subspecies *lactis* DN223 deposited under the accession No. DSM 11036. This strain has the Pfl⁻ Ldh⁻ phenotype and is derived from a parent strain which is defective in the *pfl* gene and thus incapable of growing under anaerobic conditions in the absence of acetate, i.e. having the Pfl⁻ phenotype. The mutant strain derived from the Pfl⁻ phenotype parent strain is, under the same conditions, incapable of growth even in the presence of acetate.

The above method for isolating a double mutant with the Pdc⁻ Ldh⁻ phenotype comprises in a first step the provision of the above DN223 strain, followed by a mutagenization treatment and subsequently selecting a mutant which under aerobic conditions has a growth requirement for acetate even in the presence of lipoic acid, i.e. having the Pdc⁻ Ldh⁻ Pfl⁻ phenotype. From this resulting triple mutant, a mutant is subsequently selected which has regained its capability to grow anaerobically with or without acetate, i.e. having the Pfl⁺ Ldh⁻ Pdc⁻ phenotype.

In a further aspect, the invention relates to a method of isolating a lactic acid bacterial DNA sequence coding for at least one gene product of the pyruvate dehydrogenase complex, comprising isolating a lactic acid bacterial *pdc* mutant, and identifying in a lactic acid bacterium a gene that can restore the defective Pdc activity upon transformation of said mutant and isolating the gene.

In one useful embodiment of the above method, the method comprises the steps of isolating DNA from a wild-type strain and producing a DNA library therefrom, transforming a *pdc* mutant strain with said library, selecting on a medium containing lipoic acid but without acetate a transformant strain that is capable of growing on said medium, and isolating the gene from the selected transformant.

Furthermore, the present invention relates to a method of constructing a lactic acid bacterial strain according to the invention, the method comprising replacing a wild-type *pdc* gene of a lactic acid bacterium by the above mutated gene.

Any of the above mutants or variants are potentially useful in the production of food products and accordingly, the invention relates in a further aspect to a method of producing a food product which method comprises that a culture of a lactic acid bacterium as described herein is added to the food product starting materials which are then kept under conditions appropriate for the bacteria to grow and/or to be metabolically active. One specific embodiment of the invention is where at least one further lactic acid bacterial strain is added to the starting materials. The purpose of the addition of the lactic acid bacteria depends on the food product. In some instances, a lactic acid bacterium according to invention is used to provide an increased production in the food product, such as e.g. a dairy product, of a particularly desirable aroma compound, such as diacetyl or acetoin or a precursor for such compounds. Other examples of food products where use of the present mutant strains is contemplated include meat products, vegetables, bakery products and wine.

It will also be understood that the presently provided strains will be highly useful as production strains in the manufacturing of lactic acid bacterial metabolite compounds including the above aroma compounds. Accordingly, the invention encompasses in a still further aspect a method of producing a lactic acid bacterial metabolite. Such a method comprises cultivating one or more of the lactic acid bacteria as disclosed herein in a suitable medium under industrially feasible conditions where the metabolite is produced, and isolating, if required, the metabolite from the culture. The metabolite can be isolated in accordance with any suitable conventional method of isolating the particular compound(s) from the cultivation medium. It is also possible to use the cultivation medium containing the outgrown culture of lactic acid bacteria directly as a source of one or more metabolites.

A specific example of such a production method for a lactic acid bacterial metabolite is a method of producing a diacetyl-containing flavouring product which comprises cultivating a lactic acid bacterial strain according to the invention in a suitable medium and isolating the metabolites by distillation to provide a concentrate of the metabolites. This product is e.g. used for flavouring of butter, margarine, spreads, cereal products and pop-corn. It is assumed that by using a strain according to the invention such as the strains TDH-1 or TDH-2, or a variant hereof, the product which can be obtained has a increased content of diacetyl in comparison with a diacetyl-containing product obtained by using a wild-type strain.

It is convenient to provide the lactic acid bacterium according to the invention, both when it is used as a food production strain and as a production strain for metabolites, as a lactic acid bacterial starter culture composition comprising the lactic acid bacterium selected for the specific use. Typically, such compositions contain the bacterium in concentrated form e.g. at a concentration of viable cells (colony forming units, CFUs) which is in the range of 10⁵ to 10¹³ per g of the composition such as a range of 10⁶ to 10¹² per g. Additionally, the starter culture composition may contain further components such as bacterial nutrients, cryoprotectants or other substances enhancing the viability of the bacterial active ingredient during storage. The composition can be in the form of, e.g. a frozen or freeze-dried composition.

The invention is further illustrated in the following examples and the drawing wherein:
Fig. 1 illustrates the pyruvate metabolism in lactic acid bacteria; the shown enzymatic pathways are: PFL, pyruvate formate-lyase; PDC, pyruvate dehydrogenase complex; LDH, lactate dehydrogenase; ALS, acetolactate synthetase; ILVB, second acetolactate synthetase; ALD, acetolactate decarboxylase; DR, diacetyl reductase.

### EXAMPLES

### MATERIALS AND METHODS

### 1. Bacterial strain, media and growth conditions

The following lactic acid bacterial strain was used in the examples: *Lactococcus lactis* subsp. *lactis* strain CHCC373 deposited with Deutsche Sammlung von Mikroorganismen und Zellkulturen GmbH, Mascheroder Weg 1b, D-38124 Braunschweig, Germany on 17 February 1998 under the accession No. DSM 12015.

As growth media were used: (i) the defined phosphate-buffered DN-medium (Dickely et al., 1995) with and without 2.0 g/L sodium acetate trihydrate, lipoic acid or lipoamide; and (ii) reconstituted skim milk, RSM containing 9.5% low heat skim milk powder (Milex 240 1h, MD Foods, Denmark).

The strains were cultivated at 30°C and growth and acidification were monitored by measuring pH. Anaerobic conditions for growth on agar plates were obtained by incubation in a sealed container using the Anaerocult A system (Merck, Darmstadt, Germany). In the following, anaerobic growth conditions for cultures in liquid media means cultivation without shaking and aerobic cultivation means growth under shaking.

### 2. Mutagenesis of L. lactis

A single colony of *Lactococcus lactis* subsp. *lactis* strain CHCC373 was inoculated in 10 ml DN-medium with acetate and incubated for 16 hours under vigorous shaking. To the outgrown culture 150 µl of ethyl methane sulphonate (EMS, Sigma) was added and the mixture was incubated further under shaking. After 2 hours, 10 tubes each containing 2 ml DN-medium with acetate were each inoculated with 0.2 ml of the mutagenized culture. The tubes were incubated until the following day under shaking for phenotypic expression. Sterile glycerol was added to a final concentration of 15% (v/v) and the cultures were stored at -70°C until use.

### 3. Determination of pvruvate dehydrogenase complex activity

### 3.1 Preparation of cell-free extract

A single colony of *L. lactis* was inoculated in 180 ml DN-medium with acetate as well as lipoic acid and cultivated overnight under aerobic conditions. The overnight culture was split into test tubes each containing 10 ml. After cooling for 15 min. on ice, the cells were harvested by centrifugation at 5000 rpm for 20 min. at 4°C, washed twice with 4 ml ice-cold 50 mM phosphate-buffer (pH 6.1) and resuspended in 1 ml ice-cold phosphate-buffer. The resuspended cells were transferred to a 5 ml glass tube and sonicated on ice using a Branson Sonifier 250 at the following parameters: timer, 6 min. (2 x 3 min.); duty cycle 25%; output 2. Subsequent to the sonication, the content of the tubes were transferred to ice-cold Eppendorf tubes and centrifuged at 15.000 x g for 5 min. at 4°C. Finally, the supernatants were transferred to new ice-cold Eppendorf tubes and stored at -20°C until use. Prior to use the Eppendorf tubes were thawed on ice.

### 3.2 Measurement of protein concentration of cell-free extract

For measuring the protein concentration of the cell-free extract, the Bicinchoninic acid (BCA) assay (Pierce, Rockford, USA) was used with Albumin Standard (Pierce) as protein standard.

### 3.3 Measurement of pyruvate dehydrogenase complex activity

Pyruvate dehydrogenase complex activity in the cell-free extract was measured at 30°C as described by Garrigues et al. (1997). The specific pyruvate dehydrogenase complex activity was expressed in the units of nmoles NADH produced per min. per mg of protein.

### 4. Determination of L. lactis fermentation end products

Overnight cultures of the *L. lactis* strains were inoculated in the respective media and incubated for 24 hours under the relevant growth conditions. Samples were collected and analyzed by HPLC and HS-GC for various compounds produced during the cultivation as described by Houlberg (1993, 1995a) and Richelieu et. al. (1997).

### EXAMPLE 1

### Acetate and lipoic acid requirement for aerobic growth of L. lactis

Initially, the *Lactococcus lactis* subsp. *lactis* strain CHCC373 was tested for growth on DN-medium with and without acetate and lipoic acid. The strain was streaked onto agar plates and incubated for 24 hours under anaerobic and aerobic conditions. The results are summarized in Table 1 below:

**Table 1.**

| Requirement of acetate and lipoic acid of CHCC373 | | | | |
|---|---|---|---|---|
| | - acetate | + acetate | - acetate | + acetate |
| | - lipoic acid | - lipoic acid | + lipoic acid | + lipoic acid |
| Aerobic | - | + + + | + + + | + + + |
| Anaerobic | + + + | + + + | + + + | + + + |

| | | | | |
|---|---|---|---|---|
| + + + : colony size 0.5-1 mm; | | | | |
| - : no growth after prolonged incubation | | | | |

As seen in Table 1, the strain CHCC373 has an absolute requirement for either acetate or lipoic acid under aerobic conditions. Subsequently, it was found that lipoamide could replace lipoic acid.

### EXAMPLE 2

**Isolation of Pdc defective mutants of *Lactococcus lactis* subspecies *lactis* CHCC373 and characterization hereof**

### 2.1 Isolation of mutants

A mutagenized stock of the strain CHCC373 was prepared as described above and plated in dilutions onto DN-medium agar plates containing acetate. These plates were incubated under aerobic conditions for 24 to 48 hours. Subsequently, a number of single colonies were selected and streaked onto both DN-medium agar plates containing lipoic acid but no acetate and DN-medium agar plates containing lipoic acid as well as acetate. These plates were incubated under aerobic conditions for 24 to 48 hours. Two strains designated TDH-1 and TDH-2, which in the presence of lipoic acid were unable to grow aerobically when acetate was omitted, were selected. The results for strain TDH-1 and TDH-2 are summarized respectively in Table 2 and 3 below:

**Table 2.**

| Requirement of acetate and lipoic acid of strain TDH-1 | | | | |
|---|---|---|---|---|
| | - acetate | + acetate | - acetate | + acetate |
| | - lipoic acid | - lipoic acid | + lipoic acid | + lipoic acid |
| Aerobic | - | + + + | - | + + + |
| Anaerobic | + + + | + + + | + + + | + + + |

| | | | | |
|---|---|---|---|---|
| + + + : colony size 0.5-1 mm; | | | | |
| - : no growth after prolonged incubation | | | | |

**Table 3.**

| Requirement of acetate and lipoic acid of strain TDH-2 | | | | |
|---|---|---|---|---|
| | - acetate | + acetate | - acetate | + acetate |
| | - lipoic acid | - lipoic acid | + lipoic acid | + lipoic acid |
| Aerobic | - | + + + | - | + + + |
| Anaerobic | + + + | + + + | + + + | + + + |

| | | | | |
|---|---|---|---|---|
| + + + : colony size 0.5-1 mm; | | | | |
| - : no growth after prolonged incubation | | | | |

Subsequently, it was found that strain TDH-1 and strain TDH-2 likewise were unable to grow aerobically in the presence of lipoamide when acetate was omitted.

A sample of strain TDH-1 and strain TDH-2, respectively, was deposited with Deutsche Sammlung von Mikroorganismen und Zellkulturen GmbH, Mascheroder Weg 1b, D-38124 Braunschweig, Germany on 11 February 1998 under the respective accession Nos. DSM 11996 and DSM 11997.

### 2.2 Growth and fermentation product formation of strains CHCC373, TDH-1 and TDH-2 in RSM

Single colonies of strain CHCC373, TDH-1 and TDH-2 were inoculated in 5 ml of RSM. The cultures were incubated for 24 hours under aerobic conditions. Samples were collected and analyzed for pH and for content of fermentation product formation according to the above methods. Experiments were made in triplicate.

### 2.2.1 Growth of CHCC373, TDH-1 and TDH-2

The results which are summarized in Table 4 show that strain CHCC373, TDH-1 and TDH-2 are acidifying equally well in RSM under aerobic conditions.

**Table 4.**

| End pH of CHCC373, TDH-1 and TDH-2 grown in RSM (mean ± SD) | |
|---|---|
| Strain | End pH |
| CHCC373 | 4.41 ± 0.01 |
| TDH-1 | 4.43 ± 0.01 |
| TDH-2 | 4.43 ± 0.03 |

### 2.2.2 Fermentation product formation of strain CHCC373, TDH-1 and TDH-2 in RSM under aerobic conditions

Table 5 summarizes the fermentation product formation of CHCC373, TDH-1 and TDH-2 when grown aerobically in RSM. The formation of acetate is seen to be significantly lower in RSM inoculated with either strain TDH-1 or strain TDH-2 compared with RSM inoculated with strain CHCC373. Likewise, a significantly lower production of ethanol is observed in RSM inoculated with either strain TDH-1 or strain TDH-2 compared with RSM inoculated with strain CHCC373. The concentration of acetate in RSM inoculated with either strain TDH-1 or TDH-2 is below 10% of the acetate concentration in RSM inoculated with the strain CHCC373. The concentration of ethanol in RSM inoculated with strain TDH-1 or strain TDH-2 is about 20% of the concentration in RSM inoculated with strain CHCC373. The inactivation of the pyruvate dehydrogenase complex has therefore resulted in a decreased production of both ethanol and acetate under aerobic conditions.

**Table 5.**

| Fermentation product formation for aerobic growth in RSM (mean ± SD) | | | | | | | |
|---|---|---|---|---|---|---|---|
| | AA | HAc | HMEK | ALA | DAc | EtOH | HLac |
| | ppm | ppm | ppm | ppm | ppm | ppm | g/L |
| CHCC373 | 2.2 ± | 295 ± | 1292 ± | 17.9 ± | 26.0 ± | 2.9 ± | 6.9 ± |
| | 0.1 | 1 | 67 | 1.6 | 0.5 | 1.1 | 0.1 |
| TDH-1 | 2.1 ± | 9 ± 4 | 1567 ± | 30.0 ± | 32.2 ± | 0.5 ± | 6.8 ± |
| | 0.2 | | 44 | 3.7 | 1.5 | 0.1 | 0.1 |
| TDH-2 | 2.1 ± | 21 ± 4 | 1389 ± | 25.3 ± | 28.3 ± | 0.7 ± | 7.0 ± |
| | 0.2 | | 15 | 3.5 | 0.6 | 0.2 | 0.1 |
| Abbreviations: | | | | | | | |
| AA: acetaldehyde | | | | | | | |
| HAc: acetic acid/acetate | | | | | | | |
| HMEK: acetoin | | | | | | | |
| ALA: α-acetolactate | | | | | | | |
| DAc: diacetyl | | | | | | | |
| EtOH: ethanol | | | | | | | |
| HLac: lactic acid | | | | | | | |

### 2.3 Measurement of Pdc activity

The Pdc activity of strain CHCC373, TDH-1 and TDH-2 was analyzed in accordance with the method described above. As seen in Table 6, the Pdc activity of strain TDH-1 and strain TDH-2 is below the detection limit for the assay. When a comparison is made with strain CHCC373, it is seen that strain TDH-1 and strain TDH-2 both are defective in the pyruvate dehydrogenase complex.

**Table 6.**

| Pdc activity of strain CHCC373, TDH-1 and TDH-2. | | | |
|---|---|---|---|
| Strain | CHCC373 | TDH-1 | TDH-2 |
| Specific activity of Pdc | 15.6 | < 0.5 | < 0.5 |
| % Pdc activity | 100 | < 3 | < 3 |

### REFERENCES

1. Anders, R. F., Jago, G. R. 1970. The effect of fatty acids on the metabolism of pyruvate in lactic acid streptococci. J. Dairy Res., **37**, 445-456.
2. Dickely, F., Nilsson, D., Hansen, E. B., Johansen, E. 1995. Isolation of *Lactococcus lactis* nonsense suppressors and construction of a food-grade cloning vector. Molec. Microbiol., **15**, 839-847.
3. Garrigues, C., Loubiere, P., Lindley, N. D., Cocaign-Bousquet, M. 1997. Control of the shift from homolactic acid to mixed-acid fermentation in *Lactococcus lactis:* Predominant role of the NADH/NAD⁺ ratio. J. Bacteriol., **179,** 5282-5287.
4. Gasson, M.J., Benson, K., Swindell, S., Griffin, H. 1996. Metabolic engineering of the *Lactococcus lactis* diacetyl pathway. Lait, **76**, 33-40.
5. Hinman, L. M., Blass, J. P. 1981. An NADH-linked spectrophotometric assay for pyruvate dehydrogenase complex in crude tissue homogenates. The Journal of Biological Chemistry: **256**, 6583-6586.
6. Houlberg, U. 1993. HPLC analysis: Determination of acids & carbohydrates in liquid fermentation media using internal standard. Analytical Procedure 1009, Chr. Hansen A/S.
7. Houlberg, U. 1995a. HSGC-In *situ* derivatization of acids in fermentates for physiological investigations. Technical Report 785, Chr. Hansen A/S.
8. Platteeuw, C., Hugenholtz, J., Starrenburg, M., van Alen-Boerrigter, I., De Vos, W.M. 1995. Metabolic engineering of *Lactococcus lactis:* Influence of the overproduction of α-acetolactate synthetase in strains deficient in lactate dehydrogenase as a function of culture conditions. Appl. Environ. Microbiol., **61**, 3967-3971.
9. Richelieu, M., Houlberg, U., Nielsen, J. C. 1997. Determination of α-acetolactic acid and volatile compounds by headspace gas chromatography. J. Dairy Sci., **80**, 1918-1925.
10. Snoep, J. L., de Graef, M. R., Westphal, A. H., de Kok, A., Teixeira de Mattos, M. J., Neijssel, O. M. 1993. Differences in sensitivity to NADH of purified pyruvate dehydrogenase complexes of *Enterococcus faecealis, Lactococcus lactis, Azotobacter vinelandii* and *Escherichia coli:* Implications for their activity in vivo. FEMS Microbiological Letters **114**, 279-284.

## Claims

1. A lactic acid bacterial strain which under aerobic conditions has a specific pyruvate dehydrogenase activity as determined according to Garrigues et al. (1997) which is reduced by at least 10% relative to that of the *Lactococcus lactis* subsp. *lactis* strain CHCC373 (DSM 12015) grown under the same conditions.

2. A strain according to claim 1 which under aerobic conditions has a specific pyruvate dehydrogenase activity which is at the most 12.0 nmoles NADH min⁻¹ mg⁻¹ protein.

3. A strain according to claim 1 or 2 having under aerobic conditions in RSM a production of ethanol or acetate which is at the most 80% relative to that of the *Lactococcus lactis* subsp. *lactis* strain CHCC373 (DSM 12015) grown under the same conditions.

4. A strain according to any of claims 1-3 which under aerobic conditions has a production of α-acetolactate, diacetyl, acetoin or butanediol which is increased by at least 20% relative to that of the *Lactococcus lactis* subsp. *lactis* strain CHCC373 (DSM 12015)grown under the same conditions.

5. A method of isolating a strain according to claim 1, comprising the steps of providing a lactic acid bacterial parent strain which under aerobic conditions has a growth requirement for lipoic acid and which is capable of growing in the absence of acetate, cultivating said strain on a first medium containing lipoic acid and acetate and a second medium which is the same as the first medium but without acetate and selecting a strain that is capable of growing on the first medium but not on the second medium.

6. A method of isolating a lactic acid bacterial DNA sequence coding for at least one gene product of the pyruvate dehydrogenase complex, comprising isolating a lactic acid bacterial *pdc* mutant, and identifying in a lactic acid bacterium a gene that can restore the defective Pdc activity upon transformation of said mutant and isolating the gene.

7. A method of constructing a lactic acid bacterial strain as defined in claim 1, the method comprising replacing a wild-type *pdc* gene of a lactic acid bacterium by the mutated gene of claim 6.

8. A method of producing a food product, the method comprising adding to the food product starting materials, a strain according to any of claims 1-4.

9. A method of producing a lactic acid bacterial metabolite, the method comprising cultivating a strain according to any of claims 1-4 under conditions where the metabolite is produced and, if required, isolating the metabolite from the culture medium.

10. A starter culture composition comprising a strain according to any of claims 1-4.
